Europäisches Patentamt

European Patent Office

Office européen des brevets

(19)

(11) Veröffentlichungsnummer: **0 067 319**

A2

(12) **EUROPÄISCHE PATENTANMELDUNG**

(21) Anmeldenummer: 82104305.6

(22) Anmeldetag: 17.05.82

(51) Int. Cl.³: **A 61 F 5/01**
**A 61 F 13/06**

(30) Priorität: 11.06.81 DE 3123148

(43) Veröffentlichungstag der Anmeldung:
22.12.82 Patentblatt 82/51

(84) Benannte Vertragsstaaten:
AT BE CH FR GB IT LI LU NL SE

(71) Anmelder: Hildebrandt, Hans-Dietrich, Dr. med.
Hohlesteinweg 16
D-3501 Ahnatal(DE)

(72) Erfinder: Hildebrandt, Hans-Dietrich, Dr. med.
Hohlesteinweg 16
D-3501 Ahnatal(DE)

(74) Vertreter: Freiherr von Schorlemer, Reinfried
Brüder-Grimm-Platz 4
D-3500 Kassel(DE)

(54) Knieschiene.

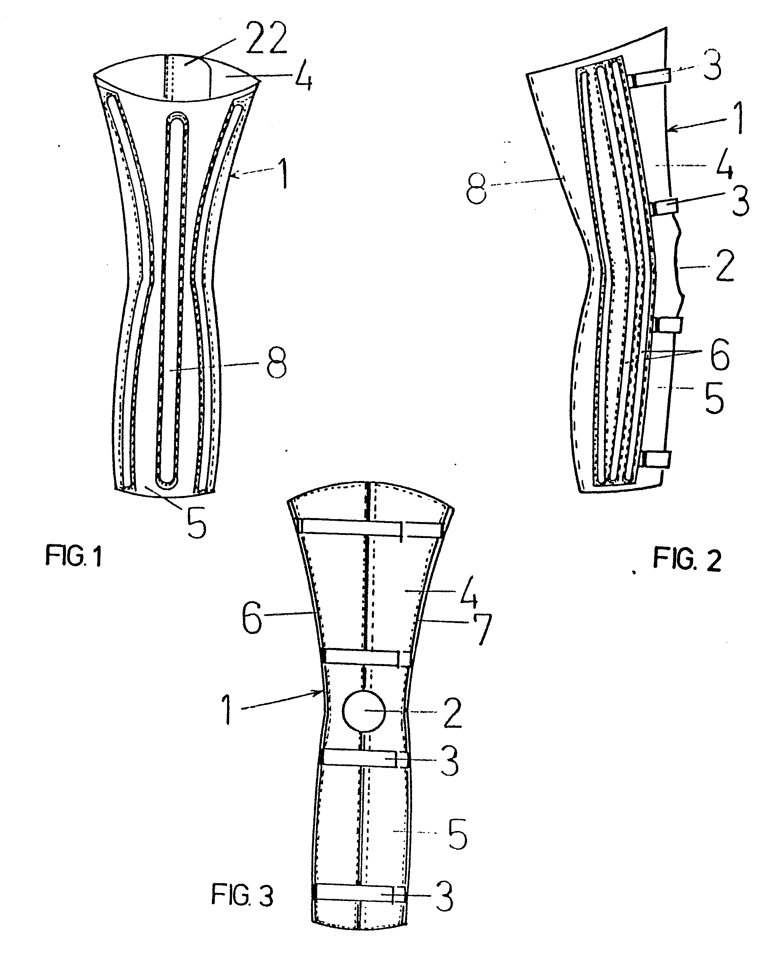

(57) Knieschiene mit einem flexiblen Mantel, der auf der Vorderseite mit verstellbaren Verschlußelementen, an den Seiten und auf der Rückseite mit Versteifungsstäben und an den seitlichen Rändern mit je einer Aussparung für die Kniescheibe versehen ist, wobei der Mantel eine an eine vorgegebene Beugestellung des Kniegelenks angepaßte Form und jeder Versteifungsstab eine zur Fixierung des Mantels und damit des Kniegelenks in der vorgegebenen Beugestellung bestimmte Krümmung aufweist (Fig. 2).

FIG. 2

Croydon Printing Company Ltd.

Knieschiene

Die Erfindung betrifft eine Knieschiene der im Oberbegriff des Anspruchs 1 definierten Gattung.

Industriell vorgefertigte Knieschienen dieser Art dienen u.a. der vorübergehenden oder auch längeren, bis zu mehreren Wochen dauernden Ruhigstellung von Kniegelenken bei Kapsel-, Band- und Meniscusverletzungen sowie Patellaluxationen. Da sie verstellbare, meistens als Klettenverschlüsse ausgebildete Verschlußelemente aufweisen, läßt sich mit ihnen der Inaktivitätsatrophie der Muskulatur und dem Schwellungsrückgang des verletzten Kniegelenks optimal Rechnung tragen, so daß sie vor allem für Langzeitbehandlungen weit besser als die grundsätzlch ebenfalls anwendbaren Gipstutoren geeignet sind.

Eine länger dauernde Fixierung mittels einer vorgefertigten Schiene ist allerdings medizinisch nur vertretbar, wenn einerseits die Vernunft des Patienten vorausgesetzt werden kann, der die Knieschiene nicht eigenmächtig entfernen, sondern allenfalls fester anziehen darf, und wenn andererseits die neuesten anatomischen und biomechanischen Verhältnisse berücksichtigt werden.

Die zuletzt genannte Voraussetzung ist mit keiner derzeit auf dem Markt befindlichen vorgefertigten Knieschiene erfüll- bar, da alle bekannten Knieschienen das Kniegelenk nur in einer nahezu gestreckten Stellung fixieren können. Derartige Knieschienen sind daher allenfalls dazu geeignet, ganz kurzfristig angelegt zu werden, beispielsweise zur Fixation eines verletzten Beins beim Transport von der Unfallstelle zum Arzt.

In neuerer Zeit hat sich nämlich ergeben, daß aus anatomischen Gründen, insbesondere zur Entlastung und somit Entstraffung der Seiten- und Kreuzbänder zwecks besserer Adaption bei deren Läsionen und zur optimalen Einstellung der Kniescheibe in deren Gleitlager, vor allem bei längerer Ruhigstellung des Kniegelenks eine Beugestellung von ca. $20^{o}$ erforderlich ist. Derartige Beugestellungen können mit den herkömmlichen Knieschienen wegen des trapezförmigen Zuschnitts ihres Mantels selbst dann nicht realisiert werden, wenn versucht würde, die vorhandenen Versteifungsstäbe entsprechend zu verbiegen.

Die Fixierung des Kniegelenks in einer Beugestellung von ca. $20^{o}$ muß daher heute ausschließlich mit einem individuell angefertigten Gipstutor vorgenommen werden. Ein solcher Gipstutor garantiert allerdings wegen der erwähnten Umfangsminderung von Oberschenkel und Kniegelenk und wegen der dadurch allmählich erfolgenden Lockerung des Verbandes insbesondere bei einer Langzeitbehandlung keine optimale Fixation des Kniegelenks.

Der Erfindung liegt die Aufgabe zugrunde, eine Knieschiene der eingangs bezeichneten Gattung mit einfachen Mitteln so weiterzubilden, daß sie das Kniegelenk in einer vorgegebenen Winkelstellung, z.B. ca. $20^{o}$, fixiert.

Zur Lösung dieser Aufgabe sind die kennzeichnenden Merkmale des Anspruchs 1 vorgesehen.

Weitere vorteilhafte Merkmale der Erfindung sind in den Unteransprüchen gekennzeichnet.

Die Erfindung bringt den Vorteil mit sich, daß sie unter Berücksichtigung der derzeitigen medizinischen Erkenntnisse das Kniegelenk in der günstigsten Beugestellung fixiert. Als Folge dieser fixierten Beugestellung ergibt sich der weitere Vorteil, daß das Rutschen der Knieschiene deutlich vermindert und somit der Tragekomfort erhöht wird. Unabhängig davon läßt sich mit Hilfe der Verschlußelemente eine eventuelle Lockerung des Verbandes korrigieren.

Die Erfindung wird nachfolgend in Verbindung mit der beiliegenden Zeichnung an einem Ausführungsbeispiel näher erläutert.

Es zeigen:

Fig. 1 bis 3 je eine Hinter-, Seiten- und Vorderansicht
einer erfindungsgemäßen Knieschiene; und

Fig. 4 fünf Zuschnitte, aus denen der Mantel der Knieschiene
nach Fig. 1 bis 3 herstellbar ist.

Die Knieschiene nach Fig. 1 enthält einen Mantel 1 aus flexiblem Material, z.B. aus Kunstleder oder einem textilen Stoff.
Wie bei herkömmlichen Knieschienen weist der Mantel 1 einen
Längsschlitz auf, der auf der Vorderseite des Beins angeordnet und längs der Seitenränder des Mantels 1 zusammengehalten wird, wobei an jedem der beiden Seitenränder eine
Aussparung vorgesehen ist, die im angelegten Zustand der Knieschiene eine im wesentlichen kreisförmige oder ovale Öffnung 2
zur Aufnahme der Kniescheibe bilden. Außerdem sind längs der
Seitenränder des Mantels 1 mehrere verstellbare Verschlußelemente 3 vorgesehen, die z.B. als Klettenverschlüsse oder
als Riemen mit entsprechenden Lochungen ausgebildet sind
und nicht nur das erstmalige Anlegen der Knieschiene, sondern auch ein späteres Festziehen der Knieschiene ermöglichen, falls sich diese beim Gebrauch aus irgendwelchen
Gründen lockern sollte.

Der Mantel 1 besitzt, wie insbesondere Fig. 2 zeigt, eine
an eine vorgegebene Beugestellung des Kniegelenks von beispielsweise 20° angepaßte Form und weist dazu einen oberhalb der Öffnung 2 angeordneten Oberschenkelabschnitt 4
und einen unterhalb der Öffnung 2 angeordneten Unterschenkelabschnitt 5 auf, deren Mittelachsen etwa in Höhe der Öffnung 2
unter dem der erwünschten Beugestellung entsprechenden Winkel
aufeinanderstoßen. Außerdem sind die Ober- und Unterschenkelabschnitte 4 und 5 an die von ihnen aufgenommenen Teile des
Ober- bzw. Unterschenkels angepaßt, um der Knieschiene eine
gute Paßform und einen guten Halt ohne Rutschgefahr zu geben.

Zur Fixierung der Form der Knieschiene beim Tragen weist der
Mantel 1 an den Seiten und an der Rückseite wenigstens je einen Versteifungsstab 6,7 bzw. 8 auf, der eine die vorgegebene Beugestellung von z.B. 20° festlegende bzw. garantierende Krümmung besitzt. Die an den Seiten befindlichen Versteifungsstäbe 6 und 7, von denen vorzugsweise je zwei vorgesehen sind, sind zu diesem Zweck, wie insbesondere Fig. 2
zeigt, im wesentlichen über die gesamte Länge des Mantels 1
erstreckt und mit einer durchgehenden Krümmung versehen.
Dagegen ist der auf der Rückseite befindliche und ebenfalls
vorzugsweise über die gesamte Länge des Mantels erstreckte
Versteifungsstab 8 einerseits entsprechend der vorgegebenen
Beugestellung gekrümmt, andererseits anatomiegerecht an
das Muskelrelief von Wade und Oberschenkel angepaßt (Fig. 2)
und dazu in seinem oberen bzw. unteren Teil in einer zum
mittleren Teil entgegengesetzten Richtung gebogen. Außerdem
ist der Versteifungsstab 8 in einer Ebene gekrümmt, die zu
den Biegeebenen der seitlichen Versteifungsstäbe 6 und 7 im
wesentlichen parallel verläuft.

Der Mantel 1 besteht gemäß einer Ausführungsform der Erfindung aus einem flexiblen und formbaren, z.B. thermoplastischen Material. Hierdurch ergibt sich der Vorteil, daß der
Mantel 1 auf eine dem gebeugten Knie nachgebildete Form
aufgezogen und dann durch Erwärmung geformt werden kann.
Seine ursprüngliche Form braucht daher nicht mit der erwünschten Form übereinstimmen, was die Herstellung wesentlich vereinfacht, da sich die aus anatomischen Gründen und
aus Gründen des Tragekomforts erwünschten Wellungen, Falten,
Ausbuchtungen od. dgl. durch thermoplastische Verformung
sehr einfach an einem einteiligen oder höchstens zweiteiligen Zuschnitt anbringen lassen.

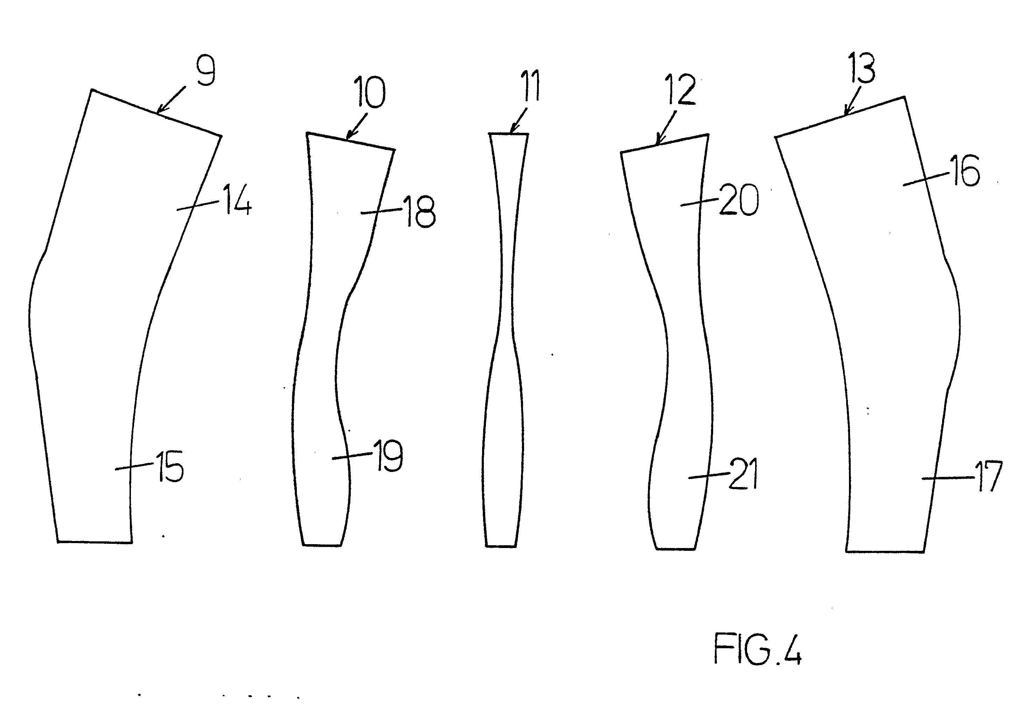

Soll der Mantel 1 dagegen aus einem flexiblen, jedoch nicht
formbaren und z.B. textilen Material hergestellt werden,
wird er zweckmäßig aus einer Mehrzahl von anatomiegerecht
geschnittenen Zuschnitten nach Fig. 4 zusammengesetzt. Gemäß
Fig. 4 sind beispielsweise fünf Zuschnitte 9 bis 13 vorgesehen, die längs ihrer Längsränder miteinander verbunden,
beispielsweise verklebt oder vernäht werden. Dabei weisen die beiden äußeren, im wesentlichen spiegelsymmetrischen Zuschnitte 9 und 13 jeweils zwei unter einem Winkel von beispielsweise 20° aneinander grenzende Schenkel 14,15 bzw. 16,17 auf, während die beiden inneren Zuschnitte 10 und 12 je zwei Schenkel 18,19 bzw. 20,21 aufweisen, die zwar ebenfalls spiegelsymmetrisch ausgebildet sind, jedoch unter einem Winkel aneinandergrenzen, der etwa der Hälfte der vorgegebenen Beugestellung, d.h. etwa 10° entspricht. Der mittlere Zuschnitt 11 schließlich besitzt im wesentlichen die Form einer Keule. Im zusammengesetzten Zustand der fünf Zuschnitte 9 bis 13 ergibt sich die Mantelform nach Fig. 1 bis 3.

Die Versteifungsstäbe 6 bis 8 bestehen aus einem Material, z.B. einem Metall, das beim Tragen der Knieschiene die Formstabilität des Mantels 1 garantiert. Geeignet sind vor allem Flach- oder Rundstäbe, die in entsprechend gekrümmte Taschen des Mantels 1 eingelegt oder vollkommen in den Mantel 1 eingenäht sind, wie die gestrichelten Linien im Bereich der Versteifungsstäbe 6 bis 8 andeuten sollen.

Zur Überdeckung von Schienbein und Oberschenkel im Bereich des durch die Verschlußelemente zusammengehaltenen Längsschlitzes des Mantels 1 sind zweckmäßig großflächige Laschen 22 (Fig. 1) aus einem weichen, polsterartigen Material vorgesehen, die an der Innenseite eines Seitenrandes des Mantels 1 angebracht sind und dessen Tragekomfort erhöhen.

Zur Berücksichtigung der verschiedenen vorkommenden Beingrößen wird die erfindungsgemäße Knieschiene zweckmäßig in einer Anzahl von Größen hergestellt und benutzt.

Die Erfindung ist nicht auf die beschriebenen Ausführungsformen beschränkt, sondern auf vielfache Weise abwandelbar. Dies gilt vor allem für die Ausbildung der Verschlußelemente, für die Ausbildung und Befestigung der Versteifungsstäbe im oder am Mantel sowie die Zahl und Form der
im Einzelfall für zweckmäßig erachteten Mantelzuschnitte.
Außer den beschriebenen Versteifungsstäben können weitere
Versteifungsstäbe zur Lagefixierung des Kniegelenks vorgesehen sein, falls sich dies als erwünscht oder notwendig
erweisen sollte.

Patentanwalt
Diplom-Physiker
**Reinfried Frhr. v. Schorlemer**

D-3500 Kassel
Brüder-Grimm-Platz 4
Telefon (0561) 15335

D 5142

Dr. Hans-Dietrich Hildebrandt, 3501 Ahnatal

Ansprüche

1) Knieschiene mit einem flexiblen Mantel, der auf der Vorderseite mit verstellbaren Verschlußelementen, an den Seiten
und auf der Rückseite mit Versteifungsstäben und an den
seitlichen Rändern mit je einer Aussparung für die Kniescheibe versehen ist, dadurch gekennzeichnet, daß der Mantel (1) eine an eine vorgegebene Beugestellung des Kniegelenks angepaßte Form und jeder Versteifungsstab (6,7,8) eine
zur Fixierung des Mantels (1) und damit des Kniegelenks in
der vorgegebenen Beugestellung bestimmte Krümmung aufweist.

2) Knieschiene nach Anspruch 1, dadurch gekennzeichnet,
daß der Mantel (1) und die Versteifungsstäbe (6,7,8) eine
zur Fixierung des Kniegelenks in einer Beugestellung von
etwa 20° bestimmte Form bzw. Krümmung aufweisen.

3) Knieschiene nach Anspruch 1 oder 2, dadurch gekennzeichnet, daß der Mantel (1) eine an die vorgegebene Beugestellung und an die angrenzenden Ober- bzw. Unterschenkelteile
angepaßte Form besitzt, an jeder Seite des Mantels (1) wenigstens ein entsprechend der vorgegebenen Beugestellung
gekrümmter Versteifungsstab (6,7) vorgesehen ist und auf
der Rückseite des Mantels (1) wenigstens ein an die vorgegebene Beugestellung und an das Muskelrelief von Wade und
Oberschenkel angepaßter Versteifungsstab (8) angeordnet
ist.

4) Knieschiene nach einem der Ansprüche 1 bis 3, dadurch
gekennzeichnet, daß der Mantel (1) aus einem thermoplastischen Material besteht.

5) Knieschiene nach einem der Ansprüche 1 bis 4, dadurch
gekennzeichnet, daß der Mantel (1) aus mehreren anatomiegerechten Zuschnitten (9 bis 13) zusammengesetzt ist.

6) Knieschiene nach Anspruch 5, dadurch gekennzeichnet,
daß der Mantel (1) aus fünf in Längsrichtung miteinander
verbundenen Zuschnitten (9 bis 13) besteht, wobei die beiden äußeren Zuschnitte (9,13) je zwei unter einem Winkel
von etwa 20$^o$ aneinander grenzende Schenkel (14,15 bzw. 16,17)
und die beiden inneren Zuschnitte (10,12) je zwei unter einem Winkel von etwa 10$^o$ aneinander grenzende Schenkel (18,19
bzw. 20,21) aufweisen und wobei der mittlere Zuschnitt (11)
etwa keulenförmig ausgebildet ist.

7) Knieschiene nach einem der Ansprüche 1 bis 6, dadurch
gekennzeichnet, daß die seitlichen Versteifungsstäbe (6,7)
aus je zwei unter einem Winkel von etwa 20$^o$ gebogenen Stäben bestehen.

8) Knieschiene nach einem der Ansprüche 1 bis 7, dadurch
gekennzeichnet, daß der rückwärtige Versteifungsstab (8)
aus einem Stab besteht, der in einer zur Biegeebene der
seitlichen Versteifungsstäbe (6,7) parallelen Ebene gebogen ist.

FIG.1

FIG. 2

FIG. 3

FIG.4